# EUROPEAN PATENT SPECIFICATION

(11) **EP 4 027 867 B1**
(45) Date of publication and mention of the grant of the patent: **28.01.2026**
(21) Application number: 20776235.2
(22) Date of filing: 11.09.2020
(51) Int. Cl.: A61B 5/00, A61B 5/0205, A61B 5/08, A61B 5/087, A61M 16/00, A61B 5/11

(54) **SYSTEMS AND METHODS FOR CONTINUOUS CARE**
SYSTEM UND VERFAHREN FÜR KONTINUIERLICHE BETREUUNG
SYSTÈMES ET PROCÉDÉS DE SOIN CONTINU

(30) Priority: 13.09.2019 US 201962899833 P
(43) Date of publication of application: 20.07.2022
(73) Proprietor: ResMed Sensor Technologies Limited, Sandyford D18 T6T7 Dublin (IE)
(72) Inventor: WREN, Michael, Dublin, D18 T6T7 (IE); SHOULDICE, Redmond, Dublin, D18 T6T7 (IE); BURRASTON, Luke, Dublin, D18 T6T7 (AU); COSTELLO, Michael John, Dublin, D18 T6T7 (IE); COFFEY, Sam, Dublin, D18 T6T7 (IE)
(74) Representative: Mathys & Squire
(86) International application number: PCT/IB2020/058479
(87) International publication number: WO 2021/048820

(56) References cited:
- WO-A1-2019/122412
- US-A1- 2005 085 738
- US-A1- 2019 110 755
- US-A1- 2019 175 858

## Description

### CROSS-REFERENCE TO RELATED APPLICATIONS

This application claims the benefit of, and priority to, U.S. Provisional Patent Application No. 62/899,833 filed on September 13, 2019.

### TECHNICAL FIELD

The present disclosure relates generally to systems and methods for determining sleep-related parameters for a user during a sleep session, and more particularly, to systems and methods for determining two or more sets of sleep-related parameters for a user during a sleep session using two or more separate and distinct sensors.

### BACKGROUND

Many individuals suffer from sleep-related respiratory disorders associated with one or more events that occur during sleep, such as, for example, snoring, an apnea, a hypopnea, a restless leg, a sleeping disorder, choking, an increased heart rate, labored breathing, an asthma attack, an epileptic episode, a seizure, or any combination thereof. These individuals are often treated using a respiratory therapy system (e.g., a continuous positive airway pressure (CPAP) system), which delivers pressurized air to aid in preventing the individual's airway from narrowing or collapsing during sleep. The respiratory therapy system can generate physiological data associated with a sleep session, which in turn can be used to determine sleep-related parameters and/or generate reports indicative of sleep quality. Exemplary methods and systems for monitoring sleep parameters during a sleep session of a subject are known from document US 2005/085738 A1. However, if the user does not use the respiratory therapy system for a portion of a sleep session (e.g., the user removes the user interface), there is a gap in the physiological data generated for the sleep session. The present disclosure is directed to solving these and other problems.

### SUMMARY

In one aspect, the invention relates to a method according to independent claim 1. Preferred implementations of the method are defined by dependent claims 2-14.

In another aspect, the invention relates to a system according to independent claim 15.

### BRIEF DESCRIPTION OF THE DRAWINGS

FIG. 1 is a functional block diagram of a system for generating physiological data associated with a user during a sleep session, according to some implementations of the present disclosure;
FIG. 2 is a perspective view of the system of FIG. 1, a user, and a bed partner, according to some implementations of the present disclosure;
FIG. 3 is a process flow diagram for a method of determining sleep-related parameters associated with a user during a sleep session, according to some implementations of the present disclosure;
FIG. 4 is a process flow diagram for a method of generating a report associated with a sleep session, according to some implementations of the present disclosure;
FIG. 5 is a process flow diagram for a method of training a machine-learning algorithm based on historical respirator device data and historical sensor data, according to some implementations of the present disclosure;
FIG. 6 illustrates an exemplary timeline for a sleep session, according to some implementations of the present disclosure; and
FIG. 7 illustrates an exemplary hypnogram associated with the sleep session of FIG. 3, according to some implementations of the present disclosure.

While the present disclosure is susceptible to various modifications and alternative forms, specific implementations and embodiments thereof have been shown by way of example in the drawings and will herein be described in detail. It should be understood, however, that the invention is not limited by the described embodiments, but it is defined by the appended claims.

### DETAILED DESCRIPTION

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB), Obstructive Sleep Apnea (OSA), apneas, Cheyne-Stokes Respiration (CSR), respiratory insufficiency, Obesity Hyperventilation Syndrome (OHS), Chronic Obstructive Pulmonary Disease (COPD), Neuromuscular Disease (NMD), and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as central apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathin. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

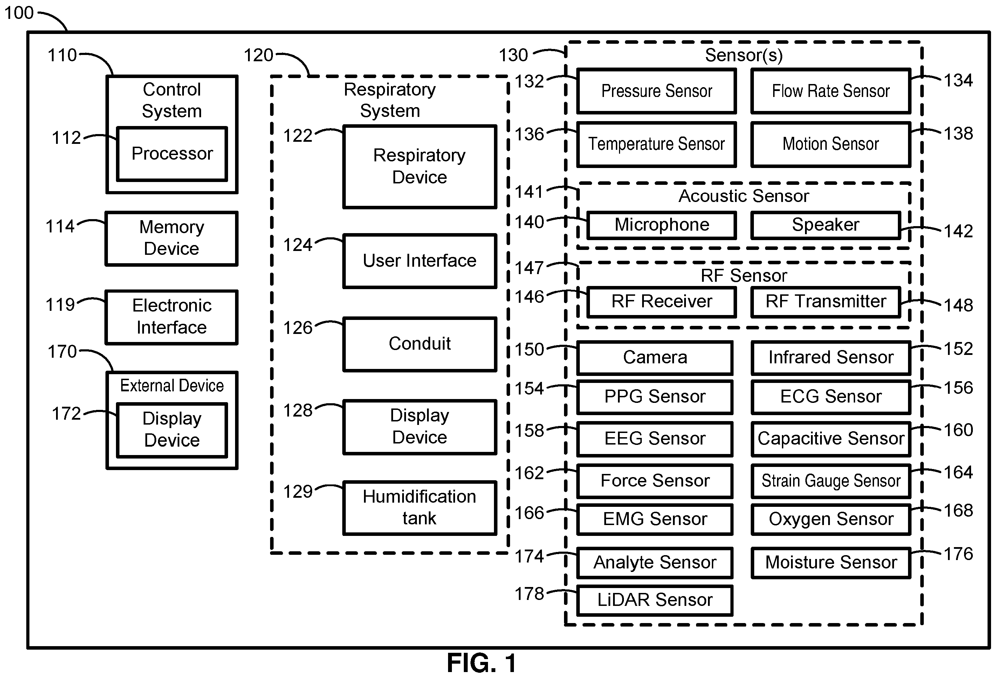

Referring to FIG. 1, a system 100 includes a control system 110, a respiratory therapy system 120, one or more sensors 130, and an external device 170. As described herein, the system 100 generally can be used to generate a first set of physiological data associated with a user during a sleep session using the respiratory system 120 and a second set of physiological data using the one or more of the sensors 130 that is external to the respiratory system 120, which in turn can be analyzed to determine a first set of sleep-related parameters and a second set of sleep-related parameters.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the external device 170, a portion (e.g., a housing) of the respiratory system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory device 122, within a housing of the external device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 (FIG. 1) stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a family history of insomnia, an employment status of the user, an educational status of the user, a socioeconomic status of the user, or any combination thereof. The medical information can include, for example, including indicative of one or more medical conditions associated with the user, medication usage by the user, or both. The medical information data can further include a multiple sleep latency test (MSLT) test result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or audio data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, over a cellular network, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the external device 170. In other implementations, the electronic interface 119 is coupled to or integrated (e.g., in a housing) with the control system 110 and/or the memory device 114.

The respiratory system 120 (also referred to as a respiratory therapy system) includes a respiratory pressure therapy device 122 (also referred to herein as respiratory device 122), a user interface 124, a conduit 126 (also referred to as a tube or an air circuit), a display device 128, and a humidification tank 129. In some implementations, the control system 110, the memory device 114, the display device 128, one or more of the sensors 130, and the humidification tank 129 are part of the respiratory device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory system 120 is generally used to treat individuals suffering from one or more sleep-related respiratory disorders (e.g., obstructive sleep apnea, central sleep apnea, or mixed sleep apnea).

The respiratory device 122 is generally used to generate pressurized air that is delivered to a user (e.g., using one or more motors that drive one or more compressors). In some implementations, the respiratory device 122 generates continuous constant air pressure that is delivered to the user. In other implementations, the respiratory device 122 generates two or more predetermined pressures (e.g., a first predetermined air pressure and a second predetermined air pressure). In still other implementations, the respiratory device 122 is configured to generate a variety of different air pressures within a predetermined range. For example, the respiratory device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory device 122 to the user's airway to aid in preventing the airway from narrowing and/or collapsing during sleep. This may also increase the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cm H₂O.

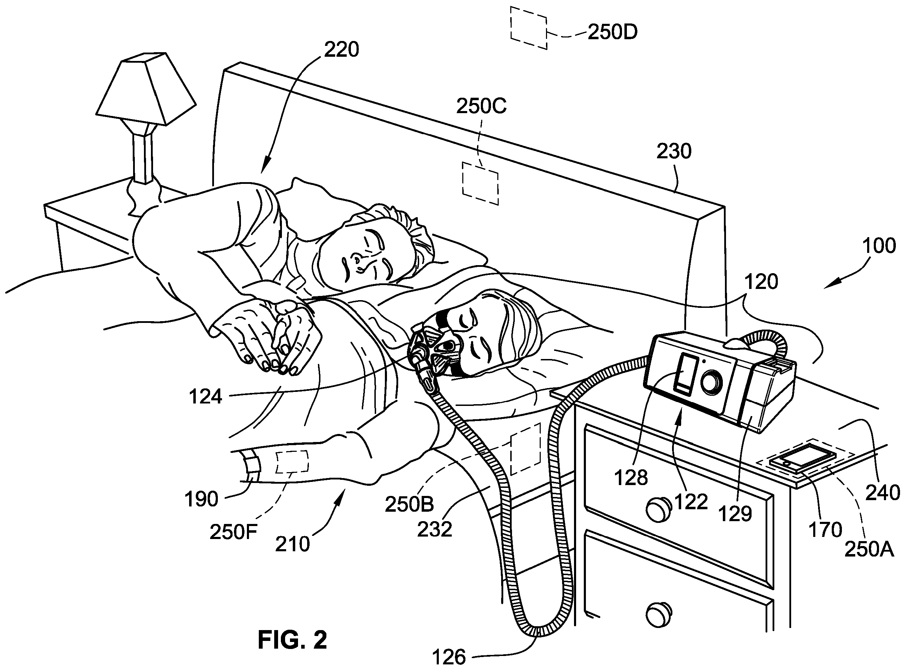

As shown in FIG. 2, in some implementations, the user interface 124 is a facial mask that covers the nose and mouth of the user. Alternatively, the user interface 124 can be a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 is a mouthpiece (e.g., a night guard mouthpiece molded to conform to the user's teeth, a mandibular repositioning device, etc.) for directing pressurized air into the mouth of the user.

The conduit 126 (also referred to as an air circuit or tube) allows the flow of air between two components of a respiratory system 120, such as the respiratory device 122 and the user interface 124. In some implementations, there can be separate limbs of the conduit for inhalation and exhalation. In other implementations, a single limb conduit is used for both inhalation and exhalation.

One or more of the respiratory device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be use, for example, to measure the air pressure and/or flow rate of pressurized air supplied by the respiratory device 122.

The display device 128 is generally used to display image(s) including still images, video images, or both and/or information regarding the respiratory device 122. For example, the display device 128 can provide information regarding the status of the respiratory device 122 (e.g., whether the respiratory device 122 is on/off, the pressure of the air being delivered by the respiratory device 122, the temperature of the air being delivered by the respiratory device 122, etc.) and/or other information (e.g., a sleep score (also referred to as a myAir score), the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory device 122.

The humidification tank 129 is coupled to or integrated in the respiratory device 122 and includes a reservoir of water that can be used to humidify the pressurized air delivered from the respiratory device 122. The respiratory device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user.

The respiratory system 120 can be used, for example, as a ventilator or a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory system 120 and a bed partner 220 are located in a bed 230 and are laying on a mattress 232. The user interface 124 (e.g., a full facial mask) can be worn by the user 210 during a sleep session. The user interface 124 is fluidly coupled and/or connected to the respiratory device 122 via the conduit 126. In turn, the respiratory device 122 delivers pressurized air to the user 210 via the conduit 126 and the user interface 124 to increase the air pressure in the throat of the user 210 to aid in preventing the airway from closing and/or narrowing during sleep. The respiratory device 122 can be positioned on a nightstand 240 that is directly adjacent to the bed 230 as shown in FIG. 2, or more generally, on any surface or structure that is generally adjacent to the bed 230 and/or the user 210.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example, physiological data, audio data, or both. Physiological data generated by one or more of the sensors 130 can be used by the control system 110 to determine a sleep-wake signal associated with a user during a sleep session and one or more sleep-related parameters. The sleep-wake signal can be indicative of one or more sleep states, including wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), or any combination thereof. The sleep-wake signal can also be timestamped to indicate a time that the user enters the bed, a time that the user exits the bed, a time that the user attempts to fall asleep, etc. The sleep-wake signal can be measured by the sensor(s) 130 during the sleep session at a predetermined sampling rate, such as, for example, one sample per second, one sample per 30 seconds, one sample per minute, etc. Examples of the one or more sleep-related parameters that can be determined for the user during the sleep session based on the sleep-wake signal include a total time in bed, a total sleep time, a sleep onset latency, a wake-after-sleep-onset parameter, a sleep efficiency, a fragmentation index, or any combination thereof.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration ratio, a number of events per hour, a pattern of events, pressure settings of the respiratory device 122, or any combination thereof. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory system 120 and/or ambient pressure. In such implementations, the pressure sensor 132 can be coupled to or integrated in the respiratory device 122. The pressure sensor 132 can be, for example, a capacitive sensor, an electromagnetic sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory device 122, an air flow rate through the conduit 126, an air flow rate through the user interface 124, or any combination thereof. In such implementations, the flow rate sensor 134 can be coupled to or integrated in the respiratory device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIG. 2), a skin temperature of the user 210, a temperature of the air flowing from the respiratory device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The microphone 140 outputs audio data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the respiratory device 122, the use interface 124, the conduit 126, or the external device 170.

The speaker 142 outputs sound waves that are audible to a user of the system 100 (e.g., the user 210 of FIG. 2). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the respiratory device 122, the user interface 124, the conduit 126, or the external device 170.

The microphone 140 and the speaker 142 can be used as separate devices. In some implementations, the microphone 140 and the speaker 142 can be combined into an acoustic sensor 141, as described in, for example, WO 2018/050913, which is hereby incorporated by reference herein in its entirety. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory device 122, the one or more sensors 130, the external device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147. In some such implementations, the RF sensor 147 includes a control circuit. The specific format of the RF communication can be WiFi, Bluetooth, or the like.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user 210 enters the bed 230 (FIG. 2), and to determine a time when the user 210 exits the bed 230.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory device 122, etc.). Thus, in some implementations, the moisture sensor 176 can be coupled to or integrated in the user interface 124 or in the conduit 126 to monitor the humidity of the pressurized air from the respiratory device 122. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the respiratory device 122, the user interface 124, the conduit 126, the humidification tank 129, the control system 110, the external device 170, or any combination thereof. For example, the microphone 140 and speaker 142 is integrated in and/or coupled to the external device 170 and the pressure sensor 130 and/or flow rate sensor 132 are integrated in and/or coupled to the respiratory device 122. In some implementations, at least one of the one or more sensors 130 is not coupled to the respiratory device 122, the control system 110, or the external device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

For example, as shown in FIG. 2, one or more of the sensors 130 can be located in a first position 250A on the nightstand 240 adjacent to the bed 230 and the user 210. Alternatively, one or more of the sensors 130 can be located in a second position 250B on and/or in the mattress 232 (e.g., the sensor is coupled to and/or integrated in the mattress 232). Further, one or more of the sensors 130 can be located in a third position 250C on the bed 230 (e.g., the secondary sensor(s) 140 is couple to and/or integrated in a headboard, a footboard, or other location on the frame of the bed 230). One or more of the sensors 130 can also be located in a fourth position 250D on a wall or ceiling that is generally adjacent to the bed 230 and/or the user 210. The one or more of the sensors 130 can also be located in a fifth position 250E such that the one or more of the sensors 130 is coupled to and/or positioned on and/or inside a housing of the respiratory device 122 of the respiratory system 120. Further, one or more of the sensors 130 can be located in a sixth position 250F such that the sensor is coupled to and/or positioned on the user 210 (e.g., the sensor(s) is embedded in or coupled to fabric or clothing worn by the user 210 during the sleep session). More generally, the one or more of the sensors 130 can be positioned at any suitable location relative to the user 210 such that the sensor(s) 140 can generate physiological data associated with the user 210 and/or the bed partner 220 during one or more sleep session.

Referring back to FIG. 1, the external device 170 includes a processor 172, a memory 174, and a display device 176. The external device 170 can be, for example, a mobile device such as a smart phone, a tablet, a laptop, or the like. The processor 172 is the same as, or similar to, the processor 112 of the control system 110. Likewise, the memory 174 is the same as, or similar to, the memory device 114 of the control system 110. The display device 176 is generally used to display image(s) including still images, video images, or both. In some implementations, the display device 176 acts as a human-machine interface (HMI) that includes a graphic user interface (GUI) configured to display the image(s) and an input interface. The display device 176 can be an LED display, an OLED display, an LCD display, or the like. The input interface can be, for example, a touchscreen or touch-sensitive substrate, a mouse, a keyboard, or any sensor system configured to sense inputs made by a human user interacting with the external device 170.

While the control system 110 and the memory device 114 are described and shown in FIG. 1 as being a separate and distinct component of the system 100, in some implementations, the control system 110 and/or the memory device 114 are integrated in the external device 170 and/or the respiratory device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for generating physiological data and determining sleep-related parameters. For example, a first alternative system includes the control system 110, the respiratory system 120, and at least one of the one or more sensors 130. As another example, a second alternative system includes the respiratory system 120, a plurality of the one or more sensors 130, and the external device 170. As yet another example, a third alternative system includes the control system 110 and at a plurality of the one or more secondary sensors 140. Thus, various systems for determining sleep-related parameters associated with a sleep session can be formed using any portion or portions of the components shown and described herein and/or in combination with one or more other components.

In many cases, a user of the respiratory system 120 will only wear the user interface 124 for a portion of a sleep session (e.g., 1 hour of an eight-hour sleep session, 2 hours of an eight-hour sleep session, 4 hours of an eight-hour sleep session, 6 hours of an eight-hour sleep session, etc.). For example, the user may initially fall asleep wearing the user interface 124 mask, wake up and remove the user interface 124, then fall back asleep. In other cases, the user may not wear the user interface 124 at all during a sleep session (e.g., the user is only using the respiratory system 120 every other day). Any sensor(s) in the respiratory system 120 cannot obtain physiological data (e.g., for determining sleep-related parameters or generating reports indicative of sleep quality) when the user does not actively use the respiratory system 120. In other words, failing to use the respiratory system 120 for the entirety of a sleep session creates a gap in the physiological data for that sleep session (or no physiological data at all), leaving users, their treating physicians, and other stakeholders (e.g., family members, bed partners, etc.) without an indication of the quality of sleep (or lack thereof) when the user is not adhering to their prescribed usage of the respiratory system 120. Typically, a user will experience worse quality sleep without the respirator system 120 than when using the system as prescribed. Determining and quantifying this difference in sleep quality is useful to encourage and/or incentivize users to utilize the system and adhere to their prescribed usage.

The same, or similar, types of physiological data that are obtained using sensors physically coupled to or integrated in the respiratory system 120 can also be generated or obtained using an independent, external sensor (e.g., a sensor integrated in a mobile device that is separate and distinct from the respiratory system 120). Advantageously, this independent sensor can be used to record physiological data during a sleep session even when the respiratory system 120 is not in use and/or powered off. However, due to variations in the sensor instrumentation, the physiological data obtained or generated by the sensor can vary from the physiological data obtained or generated by the respiratory therapy system. For example, while the respiratory therapy system may use a pressure sensor and/or flow rate sensor to determine a respiration signal or rate of the user, the external sensor may use a microphone to determine the respiration rate. Because of variations in the sensors, it is often difficult to make an accurate comparison between physiological data obtained by the respiratory therapy system and physiological data obtained by the independent sensor(s) for purposes of determining sleep-related parameters and/or generating reports indicative of sleep quality.

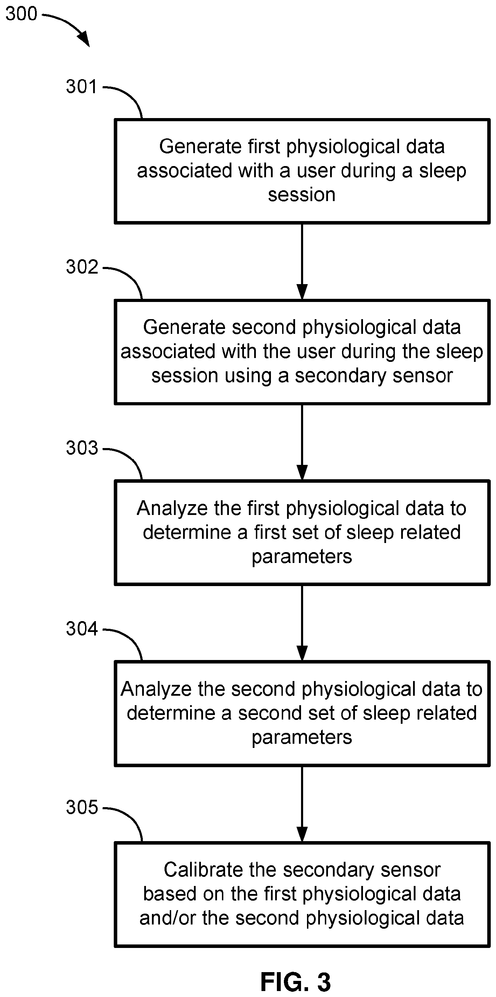

Referring to FIG. 3, a method 300 for determining sleep-related parameters for a user during a sleep session is illustrated. One or more of the steps of the method 300 described herein can be implemented using the system 100 (FIGS. 1 and 2).

Step 301 of the method 300 includes generating or obtaining first physiological data associated with a user during a sleep session. The first physiological data is generated by a first one of the one or more sensors 130 described herein, or a first group of the one or more sensors 130. For example, step 301 can include generating or obtaining first physiological data using the air pressure sensor 130 and/or the flow rate sensor 132, which are physically coupled to or integrated in the respiratory system 120 (FIG. 1). The first physiological data generated or obtained during step 301 using the first sensor can include any of the physiological data described herein. For example, the first physiological data can be derived from measures of air pressure (e.g., using the air pressure sensor 130), air flow (e.g., using the flow rate sensor 132), or both within the respiratory device 122, the user interface 124, the conduit 126 (FIG. 1), or any combination thereof.

Step 302 of the method 300 includes generating or obtaining second physiological data associated with the user during the sleep session. For example, step 302 can include generating or obtaining second physiological data using a second one or a second set of the one or more sensors 130 that is separate and distinct from the first sensor (step 301). The second physiological data generated or obtained during step 302 using the second sensor can be a different type of physiological data than the first physiological data (e.g., the first physiological data is derived from pressure and/or air flow rate and the second physiological data is derived from measurements of motions of the user or sound). Generally, the generation of the second physiological data during step 302 and the generation of the first physiological data during step 301 is substantially simultaneous such that the first physiological data (step 301) and the second physiological data (step 302) can be compared for at least a portion of time during the sleep session (when the user is using the respiratory system 120).

Unlike the first sensor (e.g., the pressure sensor 132 and/or the flow rate sensor 134 coupled to or integrated in the respiratory system 120) used during step 301 for generating the first physiological data, in some implementations, the sensor(s) used during step 302 for generating or obtaining the second physiological data is not coupled to or integrated in the respiratory system 120. That is, the second sensor(s) used in step 302 for the second physiological data is separate and distinct from the respiratory system 120. As described herein, the second sensor(s) can be positioned generally adjacent to the user 210 as an independent, standalone sensor (e.g., positioned or coupled to the bed 230, the mattress 232, the nightstand 240, the ceiling, etc.) or integrated in or coupled to the external device 170. Alternatively, the second sensor(s) used during step 302 can be coupled to a housing of the respiratory device 122 of the respiratory system 120. In some such alternative implementations, the second sensor(s) used during step 302 is not measuring a pressure or flow of the pressurized air produced by the respiratory system 120.

Step 303 of the method 300 (FIG. 3) includes analyzing the first physiological data (step 301) to determine a first set of sleep-related parameters for the user during the sleep session. For example, the first physiological data (step 301) can be stored in the memory device 114 of the control system 110 (FIG. 1), and machine-readable instructions stored in the memory device 114 are executed by the processor 112 to analyze the first physiological data. The first set of sleep-related parameters can include, for example, a first sleep score, a first flow signal, a first respiration signal, a first respiration rate, a first inspiration amplitude, a first expiration amplitude, a first inspiration-expiration ratio, a first number of events per hour, a first average number of events per hour, a first pattern of events, a first sleep state, first pressure settings of the respirator device 122, a first heart rate, a first heart rate variability, first movement of the user 210, or any combination thereof.

Step 304 of the method 300 (FIG. 3) includes analyzing the second physiological data (step 302) to determine a second set of sleep-related parameters for the user during the sleep session. For example, the second physiological data (step 302) can be stored in the memory device 114 of the control system 110, and machine-readable instructions stored in the memory device 114 can be executed by the processor 112 (FIG. 1) to analyze the second physiological data. The second set of sleep-related parameters can include, for example, a second sleep score, a second flow signal, a second respiration signal, a second respiration rate, a second inspiration amplitude, a second expiration amplitude, a second inspiration-expiration ratio, a second number of events per hour, a second average number of events per hour, a second pattern of events, a second sleep state, second pressure settings of the respirator device 122, a second heart rate, a second heart rate variability, second movement of the user 210, or any combination thereof. Step 303 and step 304 of the method 300 can be performed substantially simultaneously or sequentially (e.g., step 303 is completed before step 304 is initiated, step 304 is completed before step 303 is initiated, etc.)

Step 305 of the method 300 includes calibrating the second sensor(s) used to generate the second physiological data based at least in part on the first set of sleep-related parameters (step 303) and/or the second set of sleep-related parameters (step 304). Specifically, step 305 includes comparing the first set of sleep-related parameters (step 303) with the second set of sleep-related parameters (step 304). As described herein, because the first sensor(s) (e.g., the pressure sensor 132 and/or the flow rate sensor 134) are different than the second sensor(s), there may be variations between the first physiological data (step 301) and the second physiological data (step 302), which in turn cause variations between the determined first set of sleep-related parameters (step 303) and the determined second set of sleep-related parameters (step 304) at any given time during the sleep session. For example, prior to calibrating the second sensor(s), the first set of sleep-related parameters (step 303) can include a first pattern of events, a first average number of events per hour, a first respiration signal, or any combination thereof, and the second set of sleep-related parameters (step 304) can include a second pattern of events, a second average number of events per hour, a second respiration signal, or any combination thereof that do not match one or more of the first pattern of events, the first average number of events, or the first respiration signal. As another example, noise in the data from the second sensor(s) (step 302) may cause the control system 110 to determine, based on an analysis of the second physiological data, that the user has experienced is experiencing one of the events described herein even though the user has not or is not actually experiencing the event(s) (e.g., based on an analysis of the first physiological data).

In some implementations, step 305 includes modifying one or more parameters of the sensor(s) based at least in part on the comparison between the first set of sleep-related parameters (step 303) and the second set of sleep-related parameters (step 304). Modifying the one or more parameters of the second sensor(s) affects the second physiological data generated by the second sensor(s) during step 302. For example, modifying the parameters can aid in eliminating outlier data points and/or other noise in the recorded data. Analysis and/or modification of the one or more parameters can include linear and nonlinear transformation operations, resampling, template matching, auto and cross correlations, morphological processing, etc. Further, in some implementations, the analysis and/or modification of the one or more parameters can include characterizing and adjustment, followed by re-characterizing. Such characterizing and adjustment can involve reading physiological and/or other signals, or injecting known signals that can be recognized by both sensors (e.g., the first sensor and the secondary sensor(s)), and act as reference for the different sensors. The one or more sensor parameters of the second sensor(s) that can be modified include a frequency, a phase, a power, an amplitude, an intensity, modulation of signal of the sensor, a beam pattern, on and off of one or more antennas of the sensor, beam forming, a physical position of one or more antennas of the sensor, a physical position of the sensor, a spectral shape, or any combination thereof. In some implementations, the one or more sensor parameters are automatically modified by the control system 110. In other implementations, the control system 110 causes instructions or other indicia to be displayed (e.g., using the display device 176 of the external device 170, the display device 128 of the CPAP system 120, or both) to prompt the user to modify the one or more parameters (e.g., physically reposition the sensor).

In some implementations, step 305 includes modifying one or more parameters of the machine-readable instructions executed by the processor 112 of the control system 110 (FIG. 1) when analyzing the second physiological data during step 304 based at least in part on the comparison between the first set of sleep-related parameters (step 303) and the second set of sleep-related parameters (step 304). Modifying the parameters of the machine-readable instructions adjusts how the second set of sleep-related parameters are determined during step 304. For example, if a sleep-related parameter is determined based on at least two types of physiological data (e.g., motion data from the motion sensor 138 and image data from the camera 150), parameters of the machine-readable instructions stored in the memory device 114 can be modified to afford different weights to each of the types of physiological data. In some implementations, step 305 includes modifying one or more parameters of the second sensor and modifying one or more parameters of the machine-readable instructions stored in the memory device 114 of the control system 110.

Steps 301-305 of the method 300 can be repeated one or more times until one or more of the parameters in the first set of sleep-related parameters (step 303) matches or substantially matches or corresponds to one or more of the parameters in the second set of sleep-related parameters (step 304) (e.g., the second set of sleep-related parameters is within a predetermined standard deviation from the first set of sleep-related parameters). For example, in a first iteration of the method 300 where the first set of sleep-related parameters (step 303) does not match the second set of sleep-related parameters (step 304), the secondary sensor(s) 140 are calibrated during step 305 (e.g., by modifying one or more parameters of the sensor, modifying one or more parameters of the machine-readable instructions, or both). Steps 301-304 can be repeated in a second iteration of the method 300. If the first set of sleep-related parameters (step 303) still does not sufficiently match the second set of sleep-related parameters (step 304) during the second iteration, step 305 includes calibrating the second sensor(s) again by modifying the same parameters that were modified during the first iteration of the method 300, or by modifying different parameters of the second sensor(s) and/or the machine-readable instructions, or both. In this manner, the method 300 can be repeated a plurality of times (e.g., 5 times, 20 times, 50 times, 200 times, 1,000 times, etc.) at a predetermined interval (e.g., every 0.01 seconds, every 0.1 seconds, every 2 seconds, every ten seconds, every 60 seconds, every 5 minutes, every 30 minutes, every 60 minutes, etc.) until the calibration performed during step 305 causes the first set of sleep-related parameters (step 303) to match or correspond the second set of sleep-related parameters (step 304) in the following iteration(s).

In some implementations, the method 300 includes using a machine-learning algorithm to calibrate the secondary sensor(s) 140 during step 305. In such implementations, information indicative of (i) the modification(s) to the one or more parameters of the second sensor(s) and/or machine-readable instructions and (ii) the comparison between the first set of sleep-related parameters (step 303) and the second set of sleep-related parameters (step 304) is stored in the memory device 114 of the control system 110 (FIG. 1). This information can be used by the machine-learning algorithm over the course of multiple iterations of the method 300 to aid in calibrating the second sensor(s) by, for example, determining which modifications or combination of modifications to the one or more parameters has a corresponding effect on the difference between the first set of sleep-related parameters (step 303) and the second set of sleep-related parameters (step 304). If the machine-learning algorithm determines that certain sensor or machine-readable instruction parameters or parameter modifications are not affecting the difference in the sets of sleep-related parameters, these parameters are no longer modified in subsequent iterations of the method 300. Thus, using the machine-learning algorithm can reduce the number of iterations of the method 300 (parameter modifications during step 305) that are needed to calibrate the second sensor(s).

Once the second sensor(s) are calibrated using the method 300, the system 100 can continue to determine the second sleep-related parameters (step 304) even if the respiratory system 120 (FIG. 1) that includes the first sensor(s) is no longer generating the first set of physiological data (step 301), for example, via the pressure sensor 132 and/or the flow rate sensor 134. Advantageously, because the second sensor(s) has been calibrated using the method 300, the second set of sleep-related parameters will continue to provide information indicative of the sleep session that is the same as, or similar to, the first set of sleep-related parameters that would be obtained using the respiratory system 120. For example, if the user 210 (FIG. 2) were to wear the user interface 124 during a first portion of a sleep session but then remove the user interface 124 for a second portion of the sleep session, the system 100 continues to determine sleep-related parameters for the user 210 even though the respiratory system 120 is not being used throughout the entire sleep session by the user 210.

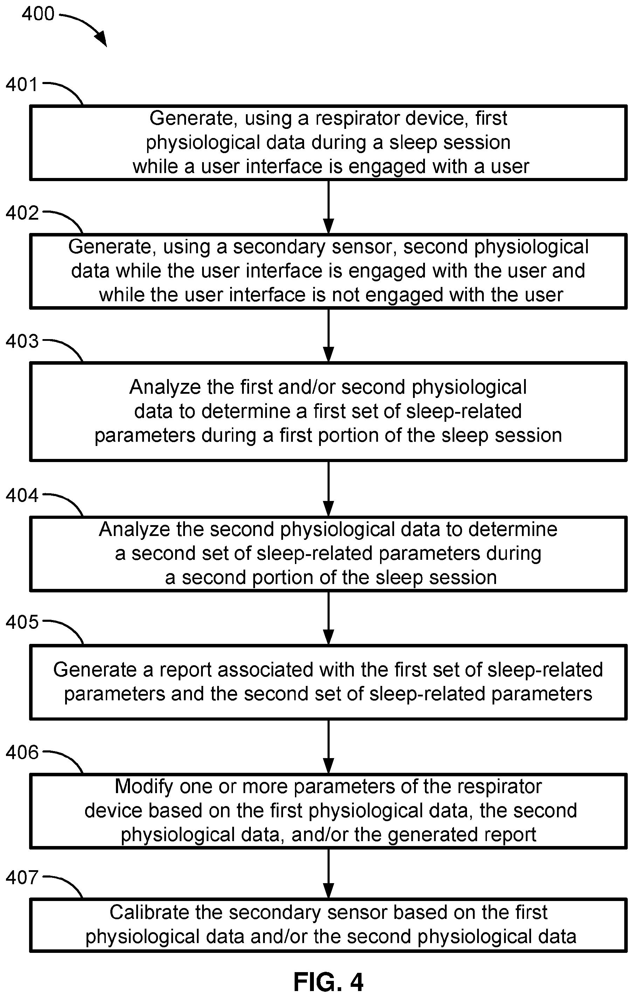

Referring to FIG. 4, a method 400 of generating a report associated with a sleep session is illustrated. One or more of the steps of the method 400 described herein can be implemented using the system 100 (FIG. 1).

Step 401 of the method 400 includes generating or obtaining first physiological data associated with a user during a sleep session while a user interface (e.g., user interface 124) is engaged with the user. Step 401 is similar to step 301 of the method 300 (FIG. 3) in that the first physiological data can be obtained using a first sensor or first sensors (e.g., the pressure sensor 132 and/or the flow rate sensor 134, which are integrated and/or coupled to the respirator device 122). Step 401 differs from step 301 (FIG. 3) in that the first physiological data is generated or obtained only when the user interface 124 of the respiratory system 120 is engaged with the user 210 (FIG. 2) during the sleep session. Thus, for portions of the sleep session where the user interface 124 is not engaged with the user 210, the first physiological data is not generated or obtained.

In some implementations, step 401 includes determining whether the user interface 124 is engaged with the user 210 using the same or different sensor(s) as the sensors used to generate the first physiological data (step 401) or the second physiological data (step 402). For example, if the pressure sensor 132 and/or the flow rate sensor 134 detect the lack of air pressure and/or air flow to the user interface 124, this can be indicative of the fact that the user interface 124 not engaged with the user 210 (FIG. 2). As another example, the mask 124 can include one or more sensors that can be used to determine whether the user interface 124 is engaged with the user 210. In a further example, image data from the camera 150 (FIG. 1) can be analyzed using an object-recognition algorithm, a facial recognition algorithm, or both, to determine whether the user interface 124 is engaged with the user 210.

Step 402 of the method 400 includes generating or obtaining second physiological data associated with the user during the sleep session while the user interface is engaged with the user and while the user interface is not engaged with the user. Step 402 is similar to step 302 of the method 300 (FIG. 3) in that the second physiological data can be obtained by at least one of the sensors 130 of the system 100 (FIG. 1) that is separate and distinct from the sensor(s) coupled to or integrated in the respiratory system 120 (e.g., a sensor that is coupled or integrated in the external device 170). In addition to using a different sensor that is not integrated in the system 120, step 402 differs from step 401 in that the second physiological data is obtained regardless of whether the user interface 124 is engaged with the user 210 (FIG. 2) during all or a portion of the sleep session.

Step 403 of the method 400 includes analyzing the first physiological data (step 401), the second physiological data (step 402), or both to determine a first set of sleep-related parameters during a first portion of the sleep session where the user interface is engaged with the user. For example, the first physiological data (step 401) and/or the second physiological data (step 402) can be stored in the memory device 114 of the control system 110 (FIG. 1), and machine-readable instructions stored in the memory device 114 are executed by the processor 112 to analyze the first and/or second physiological data. The first set of sleep-related parameters determined during step 403 are generally the same as, or similar to, the first set of sleep-related parameters in step 303 of the method 300 (FIG. 3) described herein, but differ in that the first set of sleep-related parameters determined during step 403 are limited to the first portion of the sleep session where the user interface 124 is engaged with the user 210 (FIG. 2).

Step 404 of the method 400 includes analyzing the second physiological data (step 402) to determine a second set of sleep-related parameters during a second portion of the sleep session where the user interface is not engaged with the user. For example, the second physiological data (step 402) can be stored in the memory device 114 of the control system 110 (FIG. 1), and machine-readable instructions stored in the memory device 114 are executed by the processor 112 to analyze the second physiological data. Because the first set of sleep-related parameters is limited to the first portion of the sleep session where the user interface is not engaged, the first physiological data is not used to determine the second set of sleep-related parameters during step 404.

The first portion of the sleep session (step 403) and/or the second portion of the sleep session (step 404) can include a continuous time period during the sleep session, or a combination of discontinuous time periods. For example, if the user 210 (FIG. 2) wears the user interface 124 for the first 3 hours of the sleep session, removes the user interface 124 for the next 2 hours of the sleep session, then puts the user interface 124 back on for the final 2 hours of the sleep session (totaling 7 hours), the first portion includes 5 hours and the second portion includes 2 hours. In some implementations, the first portion or the second portion comprises the entire sleep session (e.g., the user 210 wears the user interface 124 for the entire sleep session, or does not wear the user interface 124 at all during the sleep session).

Step 405 of the method 400 includes generating a report associated with the first set of sleep-related parameters (step 403) and the second set of sleep-related parameters (step 404). For example, the memory device 114 of the control system 110 (FIG. 1) can include machine-readable instructions that can be executed by the processor 112 to analyze the first set of sleep-related parameters (step 403) and the second set of sleep-related parameters (step 404) to generate the report. The generated report for the sleep session can be stored in the memory device 114 of the control system 110, displayed on the display device 176 of the external device 170 and/or the display device 128 of the respiratory system 120 subsequent to the sleep session (e.g., when the user wakes up), transmitted to a third party (e.g., a treating physician, a CPAP equipment technician), or any combination thereof. Storing the generated report in the memory device 114 allows the system 100 to compare reports over the course of a plurality of sleep sessions to determine trends and provide recommendations and/or predictions, as described further herein.

In some implementations, the report generated during step 405 can include a comparison of at least a portion of the first set of sleep related parameters (step 403) with at least a portion of the second set of sleep related parameters (step 404). In other implementations, the report can be indicative of a quality of sleep for the user 210 during the first portion of the sleep session where the user interface 124 is engaged with the user 210 and a quality of sleep for the user 210 during the second portion of the sleep session where the user interface 124 is not engaged with the user. As described herein, typically, a user will experience lower quality sleep when the user interface 124 is not engaged compared to the quality of sleep when the user interface 124 is engaged. The report can encourage adherence to prescribed usage of the respiratory system 120 by quantifying and communicating the differences in sleep quality to the user, or provide an indication that the prescribed usage of the respiratory system 120 is no longer needed.

For example, the report can include a sleep score or metric that is indicative of the quality of sleep for the first portion and the second portion of the sleep session. Generally, the sleep score can be expressed as a number (e.g., an integer) between 0 and 100, where a score of 100 is indicative of high quality sleep, a score of 0 is indicative of low quality sleep, and scores of 1-99 represent varying qualities therebetween. The sleep score can be determined based on, for example, a number or type of events during the sleep session, the duration of the sleep session, the duration of one or more sleep states during the sleep session (e.g., the relative durations of REM sleep and/or non-REM sleep), the amount of movement of the user during the sleep session, any of the other sleep-related parameters and/or events described herein, or any combination thereof. The sleep score can also be displayed or communicated to the user 210 in a manner that illustrates how the use or non-use of the respiratory system 120 has impacted the sleep score of the user.

In some implementations, the report generated during step 405 can include a sleep adherence score metric based on the prescribed usage of the respiratory system 120 for the user 210. Information indicative of the prescribed usage of the respiratory system 120 can be stored in the memory device 114 of the control system 110 (FIG. 1). The sleep adherence score metric is indicative of how closely the user 210 adhered to the prescribed usage during a sleep session. The sleep adherence score metric can be expressed as a number (e.g., an integer) between 0 and 100, as a percentage, or using other indicia (e.g., poor, fair, good, excellent, etc.). For example, if the user 210 is supposed to wear the mask 124 for at least 80% of the sleep session, and the user wears the user interface 124 for 50% of the sleep session, the sleep adherence score metric can be expressed as 62.5. The sleep adherence score metric can aid in motivating or encouraging the user 210 to adhere to their prescribed usage of the respiratory system 120 and/or provide information to a treating physician. Further details on sleep adherence score metrics (e.g., therapy quality indicator) are described in Application No. 15,520,663, filed on April 20, 2017, published as US2017/0311879, on November 2, 2017.

As described above, in some implementations, the generated report includes a sleep score or metric indicative of the quality of sleep. In such implementations, the generated report can also include a recommendation regarding an adjustment to one or more of the sleep habits described above to aid in improving the quality of the sleep score. For example, the recommendation can indicate to the user to modify the time that the user goes to bed and increase the duration of the sleep session to improve the sleep score. The report can further include a predicted quantitative improvement in the quality of the sleep score or metric corresponding to the user implementing the recommended adjustment to the one or more sleeping habits. For example, if the sleep score in the report is indicative of low quality sleep, the report can recommend increasing the sleep session duration (e.g., from 5 hours to 7 hours) and increasing the amount of time the user wear the mask 124 (e.g., from 50% of the sleep session at least 90% of the sleep session) and predict the quantitative improvement in the sleep score (e.g., an increase from a score of 50 to a score of 90). The quantitative prediction can be determined based on, for example, previously generated reports stored in the memory device 114 of the control system 110.

In some implementations, the predicted quantitative improvement in the quality of the sleep score or metric can be determined even if the user 210 has not yet used the CPAP system 120. After an individual has been diagnosed with a sleep-related respiratory disorder and prescribed a respiratory therapy system, it often takes a number of weeks or even months for the individual to obtain the respiratory therapy system. In some cases, the individual may not follow-up during this period of delay or change their mind about using the respiratory system (e.g., the user may see images of a respiratory therapy system and believe it will be too intrusive or difficult to use). Using the sensor (e.g., integrated in the external device 170) to generate the second physiological data during a sleep session allows the system 100 to determine a sleep score and predict the quantitative improvement in the quality of the sleep score if the user 210 were to use the respiratory system 120 to aid in motivating, encouraging, or incentivizing the user 210 to follow-up on the prescription and obtain and use the respiratory system 120.

In some implementations, the report generated during step 405 can include a recommendation regarding usage of the respiratory device 122 of the respiratory system 120 (FIG. 1) and/or a recommendation regarding an adjustment to sleeping habits of the user. For example, the generated report can include a recommendation to modify a time that the user goes to bed, a time that the user wakes up, a duration of the sleep session, an amount of time the user wears the user interface 124 during the sleep session (e.g., at least 33% of the duration of the sleep session, at least 66% of the sleep session, at least 75% of the sleep session, 90% of the sleep session, etc.), or any combination thereof. The recommendation regarding usage of the respirator device 122 of the CPAP system 120 can also include a recommendation to use a different respiratory system (e.g., a CPAP system that can deliver higher pressures) or different respiratory system components (e.g., a different user interface type).

The report generated during step 405 can also be used to confirm whether the respiratory system 120 is improving the quality of sleep based on the comparison between the first set of sleep-related parameters (step 303) and the second set of sleep-related parameters (step 304). For example, if the report indicates that there is little or no difference between the first set of sleep-related parameters (step 303) and the second set of sleep-related parameters (step 304), this may indicate that the user 210 no longer needs the respiratory system 120. The comparison in the report can also be used to aid in identifying other factors that may be negatively affecting the user's sleep (e.g., an undiagnosed health condition, an environmental condition, etc.) that are not being treated by the respiratory system 120.

Step 406 of the method 400 (FIG. 4) includes modifying one or more parameters of the respiratory device 122 of the respiratory system 120 based on the first physiological data (step 401), the second physiological data (step 402), the generated report (step 405), or any combination thereof. Step 406 can include, for example, a modification of a ramp time of the respiratory device 122, a modification of a pressure setting during the sleep session, a modification of a pressure setting responsive to a determination that the user has awaken from the sleep session, or any combination thereof.

Step 407 of the method 400 is the same as, or similar to, step 305 of the method 300 (FIG. 3) and includes calibrating the second sensor(s) used to generate or obtain the second physiological data during step 402. Step 407 can include modifying one or more parameters of the second sensor(s), modifying one or more parameters of the machine-readable instructions stored in the memory device 114 and executed by the processor 112 of the control system 110, or both.

While the method 400 has been described as obtaining first and second physiological data (steps 401 and 402) associated with the user 210 (FIG. 2), in some implementations, the method 400 can also include generating or obtaining third physiological data associated with the bed partner 220 (FIG. 2) during the sleep session. In such implementations, one of the one or more secondary sensors 140 and/or the respiratory system 120 can be used to generate or obtain the third physiological data for the bed partner 220, which can be stored in the memory device 114 of the control system 110. The sensor used to generate or obtain the third physiological data can be the same sensor that is used to generate or obtain the second physiological data during step 402, or a different sensor.

In implementations including the third physiological data associated with the bed partner 220, the report generated during step 405 can include a quality of sleep score or metric that is indicative of a quality of sleep for the bed partner 220 during the sleep session. Even if the bed partner 220 does not suffer from any sleep-related respiratory disorders, the bed partner 220 may have a lower sleep score than the user 210 of the respiratory system 120. For example, snoring from the user 210 may disrupt the sleep of the bed partner 220. As another example, air leakage from the user interface 124 worn by the user 210 can cause noise(s) that may disrupt the sleep of the bed partner 220. Thus, the report generated during step 405 can include a recommendation regarding an adjustment to one or more sleeping habits of the user 210 of the respiratory system 120 to aid in improving the quality of sleep metric for the bed partner 220. For example, the report can include a recommendation to increase or decrease in an average amount of time of use of the respiratory device 122 by the user 210 per sleep session. The report can also include a predicted quantitative improvement in the quality of sleep metric for the bed partner 220 corresponding to the user 210 implementing the recommended adjustment to the one or more sleeping habits.

In some implementations, the generated report can also include a recommendation on the user interface 124 worn by the user 210. For example, the report can recommend a different mask size to aid creating a better fit or interface between the mask and the user 210 or a different mask type (e.g., a nasal mask, a full mask, a cradle mask, etc.)

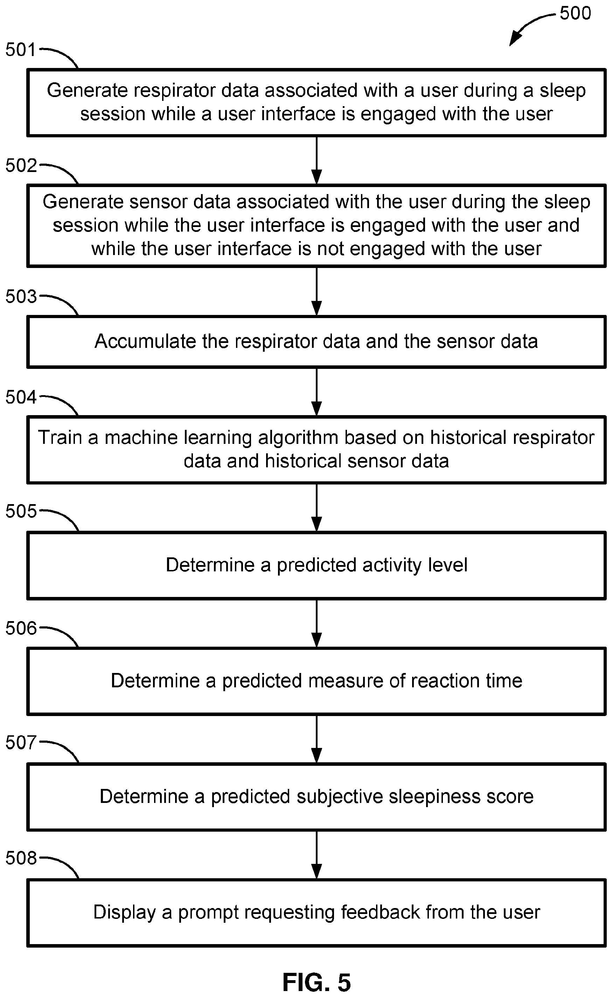

Referring to FIG. 5, a method 500 of training a machine-learning algorithm is illustrated. One or more of the steps of the method 500 described herein can be implemented using the system 100 (FIG. 1).

Step 501 of the method 500 includes generating or obtaining, using a respiratory device, respirator data associated with a user during a sleep session while a user interface is engaged with the user. Step 501 is similar to step 401 of the method 400 (FIG. 4) in that the first physiological data can be obtained using one of the one or more secondary sensors 130 (FIG. 1) of the system 100 that is integrated in the respiratory device 122 of the respiratory system 120. For example, when the user interface 124 is not engaged with the user 210 (FIG. 2), the pressure sensor 132 and/or the air flow rate sensor 134 in the respiratory device 122 does not generate or obtain the first physiological data. Like step 401 of the method 400 (FIG. 4), in some implementations, step 501 includes determining whether the user interface 124 is engaged with the user 210.

Step 502 of the method 500 includes generating or obtaining sensor data associated with the user during the sleep session while the user interface is engaged with the user and while the user interface is not engaged with the user. Step 502 is similar to step 402 of the method 400 (FIG. 4) in that the second physiological data can be obtained by a different one or more of the sensors 130 of the system 100 (FIG. 1) that is separate and distinct from the sensor(s) coupled to or integrated in the respiratory system 120 (e.g., a sensor that is coupled or integrated in the external device 170).

Step 503 of the method 500 includes accumulating respirator data and sensor data. The accumulated respirator data includes the first physiological data that is currently being generated or obtained during step 501 (hereinafter, current respirator data) and previously recorded first physiological data from prior iterations of the method 500 (hereinafter, historical respirator data). Similarly, the accumulated sensor data includes the second physiological data that is currently being generated or obtained during step 502 (hereinafter, current sensor data) and previously recorded second physiological data from prior iterations of the method 500 (hereinafter, historical sensor data). The historical respirator data and the historical sensor data can be generated over the course of multiple sleep sessions and can be stored in the memory device 114 of the control system 110 (FIG. 1). The historical respirator data and/or the historical sensor data can be stored in the memory device 114 indefinitely or for a predetermined time period (e.g., one week, one month, six months, one year, three years, etc.) and then automatically deleted.

Step 504 of the method 500 includes training a machine-learning algorithm (MLA) using the historical respirator data and the historical sensor data accumulated during step 503. The MLA is trained such that the MLA can receive as an input the current respirator data (step 501) and the current sensor data (step 502) and determine as an output a predicted activity level, a predicted measure of reaction time, a predicted subjective sleepiness sleep, or any combination thereof. That is, the MLA is trained using the historical respirator data and the historical sensor data as a training data set. The historical respirator data and the historical sensor data can be continuously accumulated or updated (step 503) to update the training data set for the MLA. The MLA can be, for example, a deep learning algorithm or a neural network, and can be stored as machine-readable instructions in the memory device 114 of the control system 110 that can be executed by the processor 112.

Step 505 of the method 500 includes determining a predicted activity level that the user will experience during a predetermined time period following the sleep session. After the MLA is trained during step 504 using historical respirator and sensor data, the MLA can receive as an input the current respirator data (step 501) and/or the current sensor data (step 502) and determine the predicted activity level for the predetermined period as an output. The predetermined period can be about between about 0.1 hours and about 24 hours after the end of the sleep session, between about 3 hours and about 16 hours after the sleep session, between about 8 hours and about 14 hours after the sleep session, about 12 hours after the sleep session, between about 24 hours and about 1 week after the sleep session, between about 12 hours and about 1 month after the sleep session, etc.

When the user 210 adheres to their prescribed usage of the respiratory therapy system, typically, the user 210 will experience better quality sleep, leading to more activity throughout the day (e.g., number of steps), a lower resting heart rate, lower heart rate variability, weight loss (e.g., by burning more calories), improved diet (e.g., consuming fewer calories), fewer headaches, or any combination thereof. The predicted activity level determined during step 505 using the trained MLA (step 504) can include a predicted number of steps, a predicted number of burned calories, a predicted resting heart rate, a predicted heart rate variability, a predicted number of headaches, a predicted weight loss, or any combination thereof during the predetermined period (e.g., 12 hours after the end of the sleep session). The predicted activity level determined during step 505 can be displayed or communicated to the user 210 using the display device 176 of the mobile device, the display device 128 of the respiratory system 120, or both. Communicating the determined predicted activity level to the user 210 can aid in encouraging or incentivizing the user 210 to adhere to their prescribed usage of the respiratory system 120.

Step 506 of the method 500 includes determining a predicted measure of reaction time to a standardized test that the user will experience subsequent to the sleep session using the trained MLA (step 504) during the predetermined time period. Typically, an individual who experienced higher quality sleep will be more alert and less sleepy than an individual that experienced lower quality sleep. Reaction time measures or assesses an individual's quickness to react to a stimulus using a standardized test such as, for example, a click reaction time test, a tap reaction time test, a speed test, a recognition test, a resolution test, a processing test, a decoding test, a reaction stick test, a light board reaction test, or any combination thereof. The predicted reaction time determined during step 506 can be displayed or communicated to the user 210 using the display device 176 of the mobile device, the display device 128 of the respiratory system 120, or both. Communicating the determined predicted reaction time to the user 210 can aid in encouraging or incentivizing the user 210 to adhere to their prescribed usage of the respiratory system 120.

Step 507 of the method 500 includes determining a predicted subjective sleepiness score that the user will experience subsequent to the sleep session using the trained MLA (step 504). The subjective sleepiness score is based on the subjective feeling of the user 210 after the sleep session and can be expressed as a number (e.g., an integer) between 0 and 100 or using other indicia (e.g., extremely or very sleepy, moderately sleepy, neutral, not sleepy, etc.). The predicted subjective sleepiness score determined during step 507 can be displayed or communicated to the user 210 using the display device 176 of the mobile device, the display device 128 of the respiratory system 120, or both. Communicating the predicted subjective sleepiness score to the user 210 can aid in encouraging or incentivizing the user 210 to adhere to their prescribed usage of the respiratory system 120.

Step 508 of the method 500 includes displaying a prompt requesting subject feedback from the user regarding a subjective feeling of the user. The subjective feeling of the user can be expressed using, for example, a descriptive term (e.g., poor, fair, neural, good, excellent, sleepy, tired, rested, alert, etc.), a number (e.g., an integer between 0 and 10, where 10 is indicative of the highest subjective feeling and 0 is indicative of the lowest subjective feeling), or both. The prompt can be displayed using the display device 176 of the external device 170 (FIG. 1), the display device 128 of the respiratory system 120, or both. The prompt generally instructs the user 210 to provide subject feedback and can be displayed or communicated visually to the user 210 as alphanumeric text and/or other indicia. Alternatively, the prompt can be communicated audibly to the user 210 (e.g., using a speaker coupled to or integrated in the external device 170 that is the same as, or similar to, the speaker 148).

Similarly, the subject feedback from the user 210 can be received by the display device 176 of the external device 170. For example, the requested subject feedback can be provided by selecting (e.g., clicking or tapping) one or more indicium displayed on the display device 176, by inputting alphanumeric text (e.g., using a touch keyboard displayed on the display device 176), using speech to text (e.g., using a microphone coupled to or integrated in the external device 170 that is the same as, or similar to, the microphone 146), or any combination thereof.

The MLA described herein can be trained during step 504 using the subject feedback provided during step 508 in response to the prompt. The subject feedback provided over the course of multiple sleep sessions can be stored in the memory device 114 of the control system 110 (FIG. 1) as historical subject feedback for training the MLA in the same or similar manner as the historical respirator data and the historical sensor data described above. The MLA can be trained to determine a quality of life metric (e.g., expressed as a numerical value or descriptive text), which is a combination of the sleep score or metric described herein and the subject feedback provided during step 508.

While the method 300, the method 400, and the method 500 have been described herein as collecting physiological data during a sleep session, more generally, these methods can be implemented (e.g., using the system 100) to generate or obtain the same or similar physiological data when the user is awake throughout the day. In such implementations, the sensor for generating the physiological data can be coupled to or integrated in the external device 170 that can be generally positioned or located adjacent to the user 210 throughout at least a portion of the day (e.g., in a pocket of the user 210). The physiological data generated or obtained for the user outside of a sleep session can be compared to the physiological data generated or obtained during the sleep session, or compared to previously recorded physiological data generated or obtained outside of a sleep session. These comparisons can be used, for example, to train the MLA during step 504 of the method 500 (FIG. 5) to predict the activity level (step 505), predict a measure of reaction time (step 506), predict a subjective sleep score (step 507), or any combination thereof.

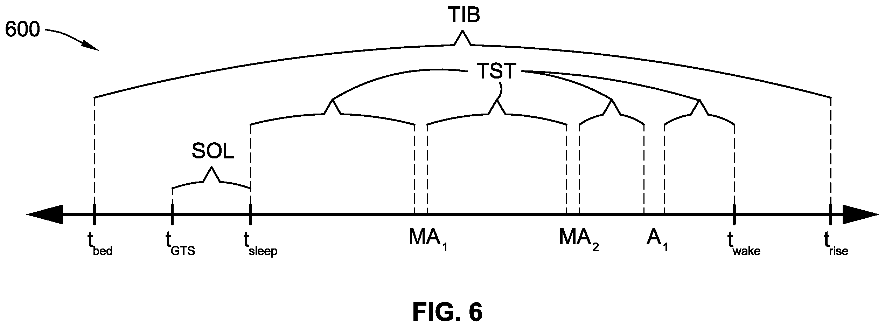

As used herein, a sleep session can be defined in a number of ways based on, for example, an initial start time and an end time. Referring to FIG. 6, an exemplary timeline 600 for a sleep session is illustrated. The timeline 600 includes an enter bed time (t_{bed}), a go-to-sleep time (t_{GTS}), an initial sleep time (tₛₗₑₑₚ), a first micro-awakening MA₁ and a second micro-awakening MA₂, a wake-up time (t_{wake}), and a rising time (tᵣᵢₛₑ).

As used herein, a sleep session can be defined in multiple ways. For example, a sleep session can be defined by an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, September 7, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, September 8, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the external device 170 (FIG. 1) to manually initiate or terminate the sleep session.

Referring to FIG. 6, the enter bed time t_{bed} is associated with the time that the user initially enters the bed (e.g., the bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the external device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (t_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 600 of FIG. 6, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (t_{GTS}) and ending at the wake-up time (t_{wake}). In some implementations, a sleep session is defined as starting at the go-to-sleep time (t_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

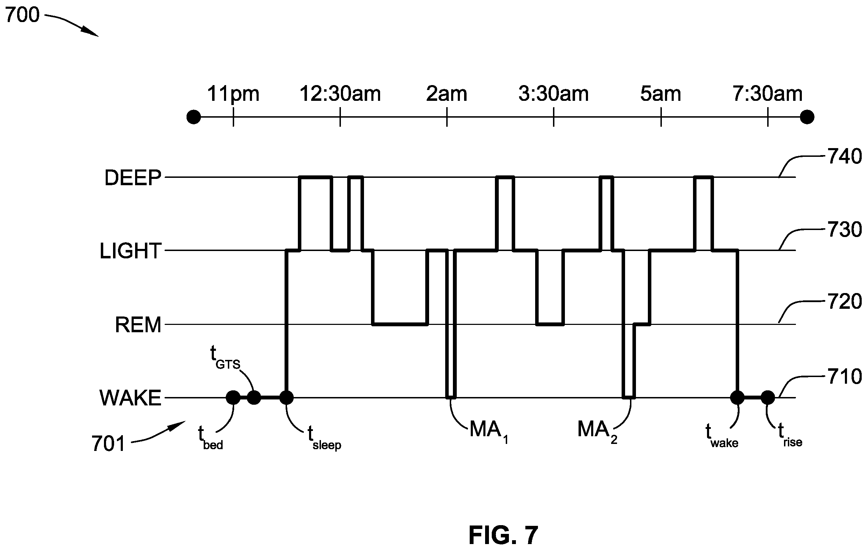

Referring to FIG. 7, an exemplary hypnogram 700 corresponding to the timeline 600 (FIG. 6), according to some implementations, is illustrated. As shown, the hypnogram 700 includes a sleep-wake signal 701, a wakefulness stage axis 710, a REM stage axis 720, a light sleep stage axis 730, and a deep sleep stage axis 740. The intersection between the sleep-wake signal 701 and one of the axes 710-740 is indicative of the sleep stage at any given time during the sleep session.

The sleep-wake signal 701 can be generated based on physiological data associated with the user (e.g., generated by one or more of the sensors 130 described herein). The sleep-wake signal can be indicative of one or more sleep states, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 700 is shown in FIG. 7 as including the light sleep stage axis 730 and the deep sleep stage axis 740, in some implementations, the hypnogram 700 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnograph 700 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (t_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 7), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the external device 170 (e.g., data indicative of the user no longer using the external device 170), data from the pressure sensor 132 and/or the flow rate sensor 134 (e.g., data indicative of the user turning on the respiratory device 122, data indicative of the user donning the user interface 124, etc.), or any combination thereof.

The invention is defined by the appended claims.

## Claims

1. A computer implemented method comprising:
receiving, from a first sensor (130a), first physiological data associated with a sleep session of a user;
receiving, from a second sensor (130b), second physiological data associated with a sleep session of a user;
determining a first set of sleep-related parameters associated with the sleep session of the user based at least in part on the first physiological data;
determining a second set of sleep-related parameters associated with the sleep session of the user based at least in part on the second physiological data;
calibrating the second sensor based at least in part on a comparison between the first set of sleep-related parameters and the second set of sleep-related parameters, wherein the calibrating the second sensor includes modifying one or more parameters of the second sensor; and
wherein the calibrated second sensor generates the second physiological data to determine the second set of sleep-related parameters even when the first sensor is not generating the first physiological data, and wherein information provided from the second set of sleep-related parameters is the same as, or similar to, information that would be obtained from the first set of sleep-related parameters.

2. The method of claim 1, wherein the first sensor is physically coupled to or integrated in a respiratory therapy system including a respiratory device (120) configured to supply pressurized air and a user interface (124) coupled to the respiratory device (120) via a conduit (126).

3. The method of claim 2, wherein the first sensor is a pressure sensor (132) or a flow rate sensor (134) and the second sensor is a motion sensor (138), an acoustic sensor (141), an RF sensor (147), a PPG sensor (154), or any combination thereof.

4. The method of claim 2 or claim 3, wherein the first sensor is configured to generate the first physiological data when the user interface is engaged with the user and the second sensor is configured to generate the second physiological data when the user interface is engaged with the user and when the user interface is not engaged with the user.

5. The method of any one of claims 1 to 4, wherein the one or more parameters of the second sensor include a frequency, a phase, a power, an amplitude, an intensity, modulation of signal of the sensor, a beam pattern, on and off of one or more antennas of the second sensor, beam forming, a physical position of one or more antennas of the second sensor, a physical position of the second sensor, or any combination thereof.

6. The method of any one of claims 1 to 5, wherein first set of sleep related parameters includes a first respiration signal associated with the user during the sleep session and the second set of sleep related parameters includes a second respiration signal associated with the user during the sleep session, wherein, optionally, prior to the calibration of the second sensor, the second respiration signal does not match the first respiration signal and wherein subsequent to the calibration of the second sensor, the second respiration signal does match the first respiration signal.

7. The method of any one of claims 1 to 6, further comprising causing an indication of the first set of sleep related parameters, the second set of sleep related parameters, or both to be communicated to the user, wherein, optionally, the indication includes a comparison of at least a portion of the first set of sleep related parameters with at least a portion of the second set of sleep related parameters and/or, the indication is indicative of a quality of sleep for the user during a first portion of the sleep session where the user interface is engaged with the user and a quality of sleep for the user during a second portion of the sleep session where the user interface is not engaged with the user.

8. The method of claim 7, further comprising causing a recommendation to adjust one or more sleeping habits to be communicated to the user, wherein, optionally, the recommendation is to modify a time that the user goes to bed, a time that the user wakes up, a duration of the sleep session, an amount of time the user wears the mask during the sleep session, or any combination thereof.

9. The method of claim 7 or claim 8, wherein the indication includes a quality of sleep metric that is indicative of a quality of sleep for the user during the sleep session and a recommendation to adjust one or more sleeping habits of the user to aid in improving the quality of sleep metric.

10. The method of claim 9, wherein the indication further includes a predicted quantitative improvement in the quality of sleep metric corresponding to the user implementing the recommended adjustment to the one or more sleeping habits.

11. The method of claim 10, wherein the recommended adjustment to the one or more sleeping habits includes a recommendation to increase an average amount of time of use of the respiratory therapy system.

12. The method of any one of claims 1 to 11, further comprising:
receiving third physiological data associated with a bed partner of the user during the sleep session;
determining a third set of sleep-related parameters associated with the bed partner during the sleep session; and
causing an indication of at least one of the third set of sleep-related parameters to be communicated to the user, the bed partner, or both,
wherein, optionally, the indication further includes a recommendation to adjust one or more sleeping habits of the user to aid in improving the at least one of the third set of sleep-related parameters for the bed partner, and
wherein, further optionally, the indication further includes a predicted quantitative improvement in the at least one of the third set of sleep-related parameters for the bed partner corresponding to the user implementing the recommended adjustment to the one or more sleeping habits of the user.

13. The method of any one of claims 2 to 12, further comprising modifying one or more parameters of the respiratory therapy system based at least in part on the first set of sleep-related parameters, the second set of sleep-related parameters, or both, wherein, optionally, the one or more parameters include a modification of a ramp time of the respiratory device, a modification of a pressure setting of the respiratory device, a modification of a pressure setting responsive to a determination that the user has awaken from the sleep session, or any combination thereof.

14. The method of any one of claims 1 to 13, wherein the first set of sleep related parameters and/or the second set of sleep related parameters include a sleep score, a flow signal, a respiration signal, a respiration rate, an inspiration amplitude, an expiration amplitude, an inspiration-expiration ratio, a number of events per hour, a pattern of events, a sleep state, pressure settings of the respirator device, a heart rate, a heart rate variability, movement of the user, or any combination thereof, wherein, optionally, the events include central apneas, obstructive apneas, mixed apneas, hypopneas, snoring, periodic limb movement, restless leg syndrome, or any combination thereof.

15. A system (100) comprising:
a control system (110) comprising one or more processors (112); and
a memory (114) having stored thereon machine readable instructions;
wherein the control system is coupled to the memory, and the method of any one of claims 1 to 14 is implemented when the machine executable instructions in the memory are executed by at least one of the one or more processors of the control system.

## Patentansprüche

1. Computerimplementiertes Verfahren, umfassend:
Empfangen erster physiologischer Daten, die mit einer Schlafsitzung eines Benutzers in Zusammenhang stehen, von einem ersten Sensor (130a);
Empfangen von zweiten physiologischen Daten, die mit einer Schlafsitzung eines Benutzers in Zusammenhang stehen, von einem zweiten Sensor (130b);
Bestimmen eines ersten Satzes von schlafbezogenen Parametern, die mit der Schlafsitzung des Benutzers in Verbindung stehen, zumindest teilweise basierend auf den ersten physiologischen Daten;
Bestimmen eines zweiten Satzes von schlafbezogenen Parametern, die mit der Schlafsitzung des Benutzers in Zusammenhang stehen, zumindest teilweise basierend auf den zweiten physiologischen Daten;
Kalibrieren des zweiten Sensors zumindest teilweise basierend auf einem Vergleich zwischen dem ersten Satz von schlafbezogenen Parametern und dem zweiten Satz von schlafbezogenen Parametern, wobei das Kalibrieren des zweiten Sensors das Modifizieren eines oder mehrerer Parameter des zweiten Sensors beinhalten; und
wobei der kalibrierte zweite Sensor die zweiten physiologischen Daten erzeugt, um den zweiten Satz von schlafbezogenen Parametern zu bestimmen, selbst wenn der erste Sensor die ersten physiologischen Daten nicht erzeugt, und wobei die aus dem zweiten Satz von schlafbezogenen Parametern bereitgestellten Informationen mit den Informationen identisch oder diesen ähnlich sind, die aus dem ersten Satz von schlafbezogenen Parametern gewonnen werden könnten.

2. Verfahren nach Anspruch 1, wobei der erste Sensor physisch mit einem Atemtherapiesystem gekoppelt oder in dieses integriert ist, das eine Atemvorrichtung (120) beinhaltet, die zur Zufuhr von Druckluft konfiguriert ist, sowie eine Benutzerschnittstelle (124), die über eine Leitung (126) mit der Atemvorrichtung (120) gekoppelt ist.

3. Verfahren nach Anspruch 2, wobei der erste Sensor ein Drucksensor (132) oder ein Durchflussratensensor (134) ist und der zweite Sensor ein Bewegungssensor (138), ein akustischer Sensor (141), ein HF-Sensor (147), ein PPG-Sensor (154) oder eine beliebige Kombination davon ist.

4. Verfahren nach Anspruch 2 oder Anspruch 3, wobei der erste Sensor so konfiguriert ist, dass er die ersten physiologischen Daten erzeugt, wenn die Benutzerschnittstelle mit dem Benutzer in Kontakt steht, und der zweite Sensor so konfiguriert ist, dass er die zweiten physiologischen Daten erzeugt, wenn die Benutzerschnittstelle mit dem Benutzer in Kontakt steht und wenn die Benutzerschnittstelle nicht mit dem Benutzer verbunden ist.

5. Verfahren nach einem der Ansprüche 1 bis 4, wobei der eine oder die mehreren Parameter des zweiten Sensors eine Frequenz, eine Phase, eine Leistung, eine Amplitude, eine Intensität, eine Modulation des Signals des Sensors, ein Strahlmuster, das Ein- und Ausschalten einer oder mehrerer Antennen des zweiten Sensors, eine Strahlformung, eine physikalische Position einer oder mehrerer Antennen des zweiten Sensors, eine physikalische Position des zweiten Sensors oder eine beliebige Kombination davon beinhalten.

6. Verfahren nach einem der Ansprüche 1 bis 5, wobei der erste Satz von schlafbezogenen Parametern ein erstes Atmungssignal beinhaltet, das mit dem Benutzer während der Schlafsitzung assoziiert ist, und der zweite Satz von schlafbezogenen Parametern ein zweites Atmungssignal beinhaltet, das mit dem Benutzer während der Schlafsitzung assoziiert ist, wobei optional vor der Kalibrierung des zweiten Sensors das zweite Atmungssignal nicht mit dem ersten Atmungssignal übereinstimmt und wobei nach der Kalibrierung des zweiten Sensors das zweite Atmungssignal mit dem ersten Atmungssignal übereinstimmt.

7. Verfahren nach einem der Ansprüche 1 bis 6, das ferner umfasst, dass eine Anzeige des ersten Satzes von schlafbezogenen Parametern, des zweiten Satzes von schlafbezogenen Parametern oder beider an den Benutzer übermittelt, wobei optional die Anzeige einen Vergleich mindestens eines Abschnitts des ersten Satzes von schlafbezogenen Parametern mit mindestens einem Abschnitts des zweiten Satzes von schlafbezogenen Parametern beinhaltet und/oder die Anzeige eine Schlafqualität für den Benutzer während eines ersten Abschnitts der Schlafsitzung anzeigt, in dem die Benutzerschnittstelle mit dem Benutzer verbunden ist, und eine Schlafqualität für den Benutzer während eines zweiten Abschnitts der Schlafsitzung, in dem die Benutzerschnittstelle nicht mit dem Benutzer verbunden ist.

8. Verfahren nach Anspruch 7, das ferner umfasst, dass eine Empfehlung zur Anpassung einer oder mehrerer Schlafgewohnheiten an den Benutzer übermittelt wird, wobei die Empfehlung optional darin besteht, die Zeit, zu welcher der Benutzer zu Bett geht, die Zeit, zu welcher der Benutzer aufwacht, die Dauer der Schlafsitzung, die Zeit, die der Benutzer die Maske während der Schlafsitzung trägt, oder eine beliebige Kombination davon zu ändern.

9. Verfahren nach Anspruch 7 oder Anspruch 8, wobei die Anzeige eine Schlafqualitätsmetrik beinhaltet, die eine Schlafqualität für den Benutzer während der Schlafphase anzeigt, sowie eine Empfehlung zur Anpassung einer oder mehrerer Schlafgewohnheiten des Benutzers, um zur Verbesserung der Schlafqualitätsmetrik beizutragen.

10. Verfahren nach Anspruch 9, wobei die Anzeige ferner eine vorhergesagte quantitative Verbesserung der Schlafqualitätsmetrik beinhaltet, die der Umsetzung der empfohlenen Anpassung der einen oder mehreren Schlafgewohnheiten durch den Benutzer entspricht.

11. Verfahren nach Anspruch 10, wobei die empfohlene Anpassung der einen oder mehreren Schlafgewohnheiten eine Empfehlung beinhaltet, die durchschnittliche Nutzungsdauer des Atemtherapiesystems zu erhöhen.

12. Verfahren nach einem der Ansprüche 1 bis 11, ferner umfassend:
Empfangen von dritten physiologischen Daten, die mit einem Bettpartner des Benutzers während der Schlafsitzung in Zusammenhang stehen;
Bestimmen eines dritten Satzes von schlafbezogenen Parametern, die mit dem Bettpartner während der Schlafsitzung in Zusammenhang stehen; und
Veranlassen, dass eine Anzeige mindestens eines Parameters aus dem dritten Satz von schlafbezogenen Parametern an den Benutzer, den Bettpartner oder beide übermittelt wird,
wobei, optional, die Anzeige ferner eine Empfehlung zum Anpassen einer oder mehrerer Schlafgewohnheiten des Benutzers beinhaltet, um zur Verbesserung des mindestens einen Parameters aus dem dritten Satz schlafbezogenen Parametern für den Bettpartner beizutragen, und
wobei, ferner optional, die Anzeige ferner eine vorhergesagte quantitative Verbesserung des mindestens einen Parameters aus dem dritten Satz schlafbezogenen Parametern für den Bettpartner beinhaltet, die der Umsetzung der empfohlenen Anpassung der einen oder mehreren Schlafgewohnheiten des Benutzers durch den Benutzer entspricht.

13. Verfahren nach einem der Ansprüche 2 bis 12, das ferner das Modifizieren eines oder mehrerer Parameter des Atemtherapiesystems umfasst, zumindest teilweise basierend auf dem ersten Satz von schlafbezogenen Parametern, dem zweiten Satz von schlafbezogenen Parametern oder beiden, wobei optional der eine oder die mehreren Parameter beinhalten eine Modifikation einer Rampenzeit des Atemgeräts, eine Modifikation einer Druckeinstellung des Atemgeräts, eine Modifikation einer Druckeinstellung als Reaktion auf eine Feststellung, dass der Benutzer aus der Schlafsitzung erwacht ist, oder eine beliebige Kombination davon.

14. Verfahren nach einem der Ansprüche 1 bis 13, wobei der erste Satz von schlafbezogenen Parametern und/oder der zweite Satz von schlafbezogenen Parametern einen Schlafwert, ein Flusssignal, ein Atmungssignal, eine Atemfrequenz, eine Einatmungsamplitude, eine Ausatmungsamplitude, ein Einatmungs-Ausatmungs-Verhältnis, eine Anzahl von Ereignissen pro Stunde, ein Ereignismuster, einen Schlafzustand, Druckeinstellungen der Vorrichtung, eine Herzfrequenz, eine Herzfrequenzvariabilität, Bewegungen des Benutzers oder eine beliebige Kombination davon, wobei die Ereignisse optional zentrale Apnoen, obstruktive Apnoen, gemischte Apnoen, Hypopnoen, Schnarchen, periodische Beinbewegungen, das Restless-Legs-Syndrom oder eine beliebige Kombination davon beinhalten.

15. System (100) umfassend:
ein Steuersystem (110), das einen oder mehrere Prozessoren (112) umfasst; und
einen Speicher (114), auf dem maschinenlesbare Anweisungen gespeichert sind;
wobei das Steuersystem mit dem Speicher gekoppelt ist und das Verfahren nach einem der Ansprüche 1 bis 14 implementiert wird, wenn die maschinenausführbaren Anweisungen in dem Speicher von mindestens einem des einen oder der mehreren Prozessoren des Steuersystems ausgeführt werden.

## Revendications

1. Procédé mis en œuvre par ordinateur comprenant :
la réception, à partir d'un premier capteur (130a), de premières données physiologiques associées avec une session de sommeil d'un utilisateur;
la réception, à partir d'un second capteur (130b), de secondes données physiologiques associées avec une session de sommeil d'un utilisateur;
la détermination d'un premier ensemble de paramètres liés au sommeil associés avec une session de sommeil d'un utilisateur basé au moins en partie sur les premières données physiologiques ;
la détermination d'un second ensemble de paramètres liés au sommeil associés avec une session de sommeil d'un utilisateur basé au moins en partie sur les secondes données physiologiques ;
la calibration du second capteur basé au moins en partie d'une comparaison entre le premier ensemble des paramètres liés au sommeil et le second ensemble des paramètres liés au sommeil, dans lequel le calibrage du second capteur inclut la modification d'un ou plusieurs paramètres de ce second capteur ; et
dans lequel le second capteur calibré génère les secondes données physiologiques afin de déterminer le second ensemble de paramètres liés au sommeil, même lorsque le premier capteur ne génère pas les premières données physiologiques, et dans lequel l'information fournie par le second ensemble de paramètres liés au sommeil est identique ou similaire à l'information qui sera obtenue à partir du premier ensemble de paramètres liés au sommeil.

2. Procédé selon la revendication 1, dans lequel le premier capteur est physiquement couplé ou intégré à un système de thérapie respiratoire comprenant un dispositif respiratoire (120) configuré pour fournir de l'air sous pression et une interface d'utilisateur (124) couplée au dispositif respiratoire (120) par un conduit (126).

3. Procédé selon la revendication 2, dans lequel le premier capteur est un capteur de pression (132) ou un capteur de débit (134), et le second capteur est un capteur de mouvement (138), un capteur acoustique (141), un capteur RF (147), un capteur PPG (154), ou toute combinaison de ceux-ci.

4. Procédé selon la revendication 2 ou 3, dans lequel le premier capteur est configuré pour générer les premières données physiologiques lorsque l'interface utilisateur est engagée avec l'utilisateur et le second capteur est configuré pour générer les secondes données physiologiques lorsque l'interface d'utilisateur est engagée avec l'utilisateur et lorsque l'interface d'utilisateur n'est pas engagée avec l'utilisateur.

5. Procédé selon l'une quelconque des revendications 1 à 4, dans lequel un ou plusieurs paramètres du second capteur incluent une fréquence, une phase, une puissance, une amplitude, une intensité, une modulation du signal du capteur, un diagramme de faisceaux, l'activation ou la désactivation d'une ou plusieurs antennes du second capteur, la formation de faisceaux, la position physique d'une ou plusieurs antennes du second capteur, la position physique du second capteur, ou toute combinaison de ces éléments.

6. Procédé selon l'une quelconque des revendications 1 à 5, dans lequel le premier ensemble de paramètres liés au sommeil comprend un premier signal respiratoire associé à l'utilisateur pendant la session de sommeil et le second ensemble de paramètres liés au sommeil comprend un second signal respiratoire associé à l'utilisateur pendant la session de sommeil, dans lequel, éventuellement, avant le calibrage du second capteur, le second signal respiratoire ne correspond pas au premier signal respiratoire et dans lequel, après le calibrage du second capteur, le second signal respiratoire correspond au premier signal respiratoire.

7. Procédé selon l'une quelconque des revendications 1 à 6, comprenant en outre la génération d'une indication du premier ensemble de paramètres liés, le second ensemble de paramètres liés au sommeil, ou les deux à communiquer à l'utilisateur, dans lequel, éventuellement, l'indication comprend une comparaison d'au moins une partie du premier ensemble de paramètres liés au sommeil avec au moins une partie du second ensemble de paramètres liés au sommeil et/ou l'indication est représentative d'une qualité de sommeil de l'utilisateur pendant une première portion de la session de sommeil où l'interface d'utilisateur est engagée avec l'utilisateur et d'une qualité de sommeil de l'utilisateur pendant une seconde portion de la session de sommeil où l'interface d'utilisateur n'est pas engagée avec l'utilisateur.

8. Procédé selon la revendication 7, comprenant en outre la génération d'une recommandation visant à ajuster une ou plusieurs habitudes de sommeil, à communiquer à l'utilisateur, dans laquelle, éventuellement, la recommandation est de modifier l'heure à laquelle l'utilisateur se couche, l'heure à laquelle il se réveille, la durée de la session de sommeil, la durée pendant laquelle l'utilisateur porte le masque pendant de la session de sommeil, ou toute combinaison de ces éléments.

9. Procédé selon la revendication 7 ou 8, dans lequel l'indication comprend une mesure de la qualité du sommeil qui est représentative de la qualité du sommeil pour l'utilisateur pendant la session de sommeil et une recommandation visant à ajuster une ou plusieurs habitudes de sommeil de l'utilisateur pour améliorer cette mesure de qualité du sommeil.

10. Procédé selon la revendication 9, dans lequel l'indication comprend en outre une amélioration quantitative prédite de la mesure de la qualité du sommeil, correspondant à la mise en œuvre par l'utilisateur de l'ajustement recommandé d'une ou plusieurs habitudes de sommeil.

11. Procédé selon la revendication 10, dans lequel l'ajustement recommandé d'une ou plusieurs habitudes de sommeil comprend une recommandation visant à augmenter la durée moyenne d'utilisation du système de thérapie respiratoire.

12. Procédé selon l'une quelconque des revendications 1 à 11, comprenant en outre :
la réception de troisièmes données physiologiques associées au partenaire de lit de l'utilisateur pendant la session de sommeil ;
la détermination d'un troisième ensemble de paramètres liés au sommeil associés au partenaire de lit pendant la session de sommeil ; et
la provocation d'une indication d'au moins un du troisième ensemble de paramètres liés au sommeil à communiquer à l'utilisateur, au partenaire de lit, ou aux deux ;
dans lequel, éventuellement, l'indication comprend en outre une recommandation pour ajuster une ou plusieurs habitudes de sommeil de l'utilisateur afin d'améliorer au moins un du troisième ensemble de paramètres liés au sommeil du partenaire de lit ; et
dans lequel, en outre et éventuellement, l'indication comprend en outre une amélioration quantitative prédite d'au moins un du troisième ensemble de paramètres liés au sommeil du partenaire de lit, correspondant à la mise en œuvre par l'utilisateur de l'ajustement recommandé d'une ou plusieurs habitudes de sommeil.

13. Procédé selon l'une quelconque des revendications 2 à 12, comprenant en outre la modification d'un ou plusieurs paramètres du système de thérapie respiratoire, basée au moins en partie sur le premier ensemble de paramètres liés au sommeil, le second ensemble de paramètres liés au sommeil, ou les deux, dans laquelle, éventuellement, les un ou plusieurs paramètres incluent une modification du temps de montée du dispositif respiratoire, une modification d'un réglage de pression du dispositif respiratoire, une modification d'un réglage de pression en réponse à une détermination selon laquelle l'utilisateur s'est réveillé de la session de sommeil, ou toute combinaison de ces éléments.

14. Procédé selon l'une quelconque des revendications 1 à 13, dans lequel le premier ensemble de paramètres liés au sommeil et/ou le second ensemble de paramètres liés au sommeil comprend un score de sommeil, un signal de débit, un signal respiratoire, une fréquence respiratoire, une amplitude d'inspiration, une amplitude d'expiration, un rapport inspiration-expiration, un nombre d'événements par heure, un schéma d'événements, un état de sommeil, des réglages de pression du dispositif respiratoire, une fréquence cardiaque, une variabilité de la fréquence cardiaque, un mouvement de l'utilisateur, ou toute combinaison de ces éléments, dans lequel, éventuellement, les événements comprennent des apnées centrales, des apnées obstructives, des apnées mixtes, des hypopnées, des ronflements, des mouvements périodiques des membres, le syndrome des jambes sans repos, ou toute combinaison de ceux-ci.

15. Système (100) comprenant :
un système de commande (110) comprenant un ou plusieurs processeurs (112) ; et
une mémoire (114) ayant stockées des instructions lisibles par machine ;
dans lequel le système de commande est couplé à la mémoire, et le procédé selon l'une quelconque des revendications 1 à 14 est mis en œuvre lorsque les instructions exécutables par machine présentes dans la mémoire sont exécutées par au moins un des processeurs du système de commande.
